# EUROPEAN PATENT APPLICATION

(11) **EP 2 123 214 A1**
(43) Date of publication of application: **25.11.2009**
(21) Application number: 08104033.9
(22) Date of filing: 20.05.2008
(51) Int. Cl.: A61B 1/07, A61B 1/227, A61B 5/107, G01B 11/24, A61F 2/30, H04R 25/00

(54) **A probing device and a system for obtaining geometrical data related to a cavity**

(71) Applicant: Oticon A/S, 2765 Smørum (DK); Widex A/S, 3500 Værløse (DK)
(72) Inventor: Petersen, Henrik Poulin, DK-2765, Smørum (DK)

(57) **Abstract**

Disclosed is a probing device for obtaining geometrical data related to a cavity, the probing device comprising at least one light source for generating electromagnetic radiation; a handheld probe body comprising a probe tip for obtaining said geometrical data related to a cavity; at least one light guide for guiding electromagnetic radiation; wherein a first end of said at least one light guide is optically coupled to said probe tip; and wherein the at least one light source is optically coupled to a second end of said at least one light guide and wherein the at least one light source is rigidly connected to said probe body.

Thereby is achieved to remove and/or reduce light output amplitude dependence of the probing device due to motion of the probing device.

## Description

### Field of the invention

The present invention is related to a probing device and a system for obtaining geometrical data related to a cavity, such as for example the ear and ear canal of the human body.

### Background of the invention

In order to scan a cavity such as for example the ear and ear canal of the human body, a handheld device may be used to scan the cavity using light and to generate a data mapping of the internal surface of the ear and ear canal, so that 3-dimensional data or a digital model of the internal surface of the ear and ear canal can be obtained.

The handheld device requires a light source in order to scan the ear and ear canal. The light source is located in a separate external box connected to the handheld device via a bulky and expensive optical fiber.

It remains a problem to reduce and/or remove motion dependent variations in the amplitude of the light used by the handheld device to scan the cavity due to motion of the handheld device. Further, it remains a problem that the optical fibers are bulky and expensive.

### Summary

Disclosed is a probing device for obtaining geometrical data related to a cavity, the probing device comprising at least one light source for generating electromagnetic radiation; a handheld probe body comprising a probe tip for obtaining said geometrical data related to a cavity; at least one light guide for guiding electromagnetic radiation; wherein a first end of said at least one light guide is optically coupled to said probe tip; and wherein the at least one light source is optically coupled to a second end of said at least one light guide and wherein the at least one light source is rigidly connected to said probe body.

Consequently, it is an advantage that the light source is rigidly fixed in connection with the probe body because thereby a light guide connecting said light source to the tip of the probe may be substantially fixed with respect to the probe body (because both the probe tip and the light source are rigidly fixed in connection to the probing device) and thus the motion dependent variations in the amplitude of the light emitted from the probe tip may be reduced and/or removed.

In an embodiment, the at least one light source is contained in said probe body.

An advantage of this embodiment is that the light source (and thus also the light fiber) may be contained in the probe body thereby reducing risk of damaging the light source (and/or light guide).

In an embodiment, the at least one light source is thermally coupled to a heat sink.

An advantage of this embodiment is that the heat generated by the at least one light source may be dissipated to and/or absorbed by the heat sink. Thereby, the output from the light source may be more stable than without a heat sink due to removal/reducing of thermal effects in the output from light source.

In an embodiment, the probing device further comprises a control circuit for controlling said at least one light source.

An advantage of this embodiment is that the control circuit may, for example, control the modulation of an optical output from the at least one light source and/or control the power of the optical output signal from the one or more light sources. Thereby, for example, a plurality of light sources may, for example, be synchronized.

In an embodiment, the control circuit is thermally coupled to and contained in a heat sink.

An advantage of this embodiment is that by containing the control circuit in the heat sink, e.g. made of aluminum such as an aluminum box, the control circuit may be shielded from electromagnetic noise from the surroundings and likewise, the surroundings may be shielded from the electromagnetic signals generated by the control circuit. Additionally, by thermally coupling the control circuit to the heat sink, the heat generated by the control circuit may be dissipated to and/or absorbed by the heat sink thereby reducing and/or removing possible thermal generated errors in the control circuit.

In an embodiment, the at least one light source comprises at least one laser emitting light in the form of electromagnetic radiation at a wavelength chosen in the interval 400nm - 480nm.

An advantage of this embodiment is that the at least one laser thereby emits violet or blue light which may dissipate easier than, for example, green light.

In an embodiment, the at least one light guide comprises at least one multimode fiber.

An advantage of this embodiment is that optical coupling of a light source to a multimode optical fiber is easier than optical coupling of a light source to a single mode fiber because the multimode fiber may have a higher numerical aperture than a single mode fiber.

In an embodiment, each of the at least one light guides comprises a connector at their second end for optically coupling respective ones of said at least one light source to respective ones of the at least one light guide via respective housings.

An advantage of this embodiment is that using a connector on the second end of a light guides and a housing containing a light source, the optical coupling of the light guide to the light source may be expediently performed due to a predefined correlation between the output from the light source and the numerical aperture of the light guide. The connector may be frictionally coupled to said housing. A further advantage of this embodiment is enable easy replacement of the light source or the probe tip and light guide.

In an embodiment, the connector is chosen from the group consisting of SC optical connector, MU optical connector, ST optical connector, LC optical connector, FC optical connector and SMA optical connector.

The present invention relates to different aspects including the probing device described above and in the following, and a corresponding system, said system yielding one or more of the benefits and advantages described in connection with the first mentioned aspect, and said system having one or more embodiments corresponding to the embodiments described in connection with the first mentioned aspect and/or disclosed in the appended claims.

In particular, disclosed herein is a system for obtaining geometrical data related to a cavity, the system comprising at least one probing device according to the invention and the system further comprising at least a plurality of magnetic sensors for detecting the at least one probing device.

### Brief description of the drawings

The above and/or additional objects, features and advantages of the present invention, will be further elucidated by the following illustrative and nonlimiting detailed description of embodiments of the present invention, with reference to the appended drawings, wherein:
Fig. 1 shows a schematic view of an apparatus for obtaining geometrical data relating to the internal surface of a cavity.
Fig. 2 shows a schematic view of another example of an apparatus for obtaining geometrical data relating to the internal surface of a cavity.
Fig. 3 shows a sectional view of the distal end of a probe of an apparatus for obtaining geometrical data relating to the internal surface of a cavity.
Fig. 4 shows a probing device comprising a distal light-emitting end portion and a rod portion which connects the distal light-emitting end portion to a proximal part.
Fig. 5 shows an embodiment in which a second end of a light guide is connected to a light source via a connector and a housing.
Fig. 6 is a side view showing the human ear and illustrating the use of the apparatus described herein.

### Detailed description

In the following description, reference is made to the accompanying figures, which show by way of illustration how the invention may be practiced.

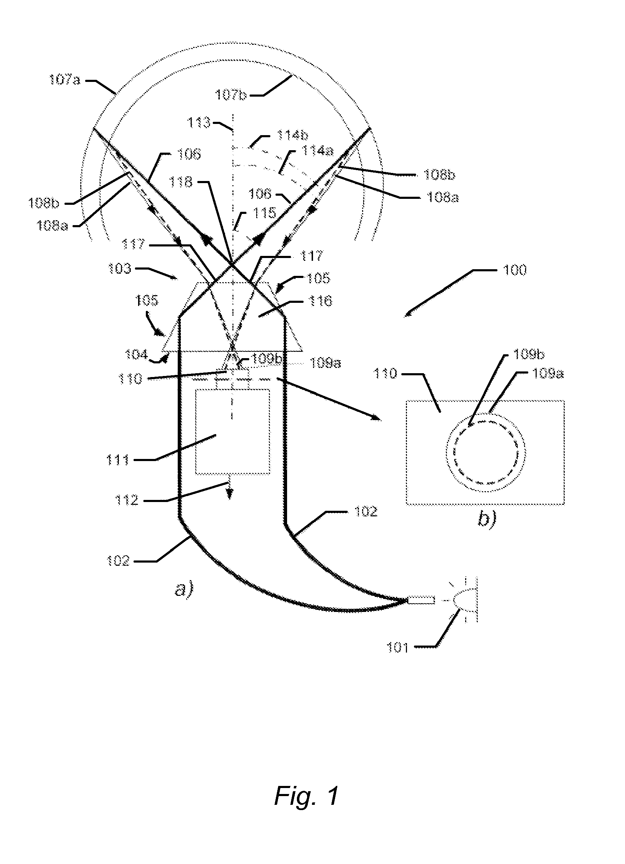

Fig. 1a shows a schematic view of an example of an apparatus for obtaining geometrical data relating to the internal surface of a cavity. The apparatus, generally designated 100, comprises a distal end portion 103 including an optical system 116 for emitting and receiving light, a light source 101 and light guides 102 for directing the light from the light source 101 to a rear surface 104 of the optical system 116. The optical system 116 may comprise one or more lenses and/or one or more reflecting surfaces 105 for directing the light from the light guides 102 as one or more beams 106 to the internal surface 107a, b of a cavity.

The light beams 106 are emitted at an acute angle 115 relative to the optical axis 113 of the optical system 116 which also defines the direction of insertion of the distal end portion 103 into the cavity. The light beams 106 are emitted from respective exit positions 117 radially displaced from the optical axis. Furthermore, the exit position 117 and the position where the light beam intersects with the cavity wall 107a, b, are positioned on opposite sides of the optical axis 113. The light beams 106 thus cross the optical axis. Furthermore, the light beams 106 intersect at a point 118 in front of the probe; in the example of fig. 1 the beams intersect with each other on the optical axis.

In the example of fig. 1, two light guides 102 are shown. However, it will be appreciated that generally a different number of light guides may be used, e.g. 3, 4, 5, 6, or even a larger number of light guides. In some embodiments a uniform illumination over a full circumference is achieved by providing a relatively large number, e.g. between 60 or 80, fibres. The fibres are divided into bundles, e.g. 6 bundles, and each bundle is illuminated with a respective light source. This gives the opportunity to make an automatic control of the emitted power at different sections of the probe. This is advantageous as it allows staying in the dynamic range of the light detector, when measuring surfaces at different distances in different sections at the same time.

For example, the light guides may be arranged such that the emitted light beams 106 intersect with each other as shown in figure 1, so as to define a double cone where the point of intersection 118 defines the apex of the cone. In the example of fig. 1, the beams 106 intersect each other on the optical axis.

The emitted light beams 106 are reflected from the internal surface 107a, b of the cavity and at least a portion of the reflected light will be reflected back in the direction of the distal end portion 103 as indicated by reflected beams 108a, b in fig. 1. The optical system 116 of the distal end portion 103 thus also functions as a light receiving system and receives the reflected light 108a, b from the cavity walls 107a, b. The optical system 116 directs the received light 108a, b towards a light sensitive element 110, e.g. an array of light sensitive elements such as a CCD or another position sensitive light detector.

Generally, when the CCD is a colour sensitive CCD element, colour information may be used when analyzing the light reflected from the surface of an ear canal. If white light is used, it is possible to determine the relative content of red, green and blue light in the received signal, and thereby foreign objects such as earwax may be identified. This is because earwax will reflect the light in other wavelength ranges than the naked skin of the ear canal.

Furthermore, the detected signal from the CCD may be displayed on a display, via an eyepiece, or the like, as a received image, thus allowing the probe described herein to be used in a fashion similar to an endoscope. Such an image may be valuable for the person conducting an ear scan, e.g. for a visual inspection of the measured cavity and/or so as to provide a visual control as to how close the probe is to the end wall, e.g. the tympanic membrane.

The light source 101 may be any suitable light source, e.g. a one or more light emitting diodes (LED) or diode lasers. LEDs provide a low noise level as they avoid noise from speckle, while diode lasers provide a high output power.

In fig. 1, the cavity surface 107a, b is shown as an example in a first distance at 107a from the tip of the probe and in a second distance at 107b closer to the tip of the probe. Light reflected from the surface at 107a will enter the optical system 116 as light 108a at an incident angle 114a relative to the optical axis, while light reflected from the surface at 107b will enter the optical system 116 as light 108b at an incident angle 114b relative to the optical axis. Consequently, the optical system 116 directs the incoming light 108a and 108b to respective positions 109a and 109b on the light sensitive element 110, thereby allowing the determination of the distance to the points 107a and 107b in the direction of the emitted light 106 from the position of the detected light on the light sensitive element 110. Fig. 1b illustrates positions of constant distance on the light sensitive area of the detector 110 for two different distances 109a and 109b respectively.

The apparatus 100 further comprises a signal analysis circuit 111 which generates an output signal 112, e.g. an analogue signal or a digital data signal. In some embodiments, the output signal is indicative of an intensity distribution of the detected light across the light sensitive area 110 of the detector. Alternatively or additionally, the signal analysis circuit may perform additional signal processing steps, e.g. including the actual distance calculation based on an incident angle determined from the detected locations 109a and 109b. The distance may be calculated by means of a conventional triangulation resulting in a distance from the probe to the locations where the beams 106 intersect with the cavity wall 107a,b, i.e. a distance in a direction having a component in a radial direction from the optical axis 113. Alternatively, the distance calculation and/or further signal processing may be performed by a separate signal/data processing unit, e.g. on a computer such as a PC to which the apparatus 100 may be connected.

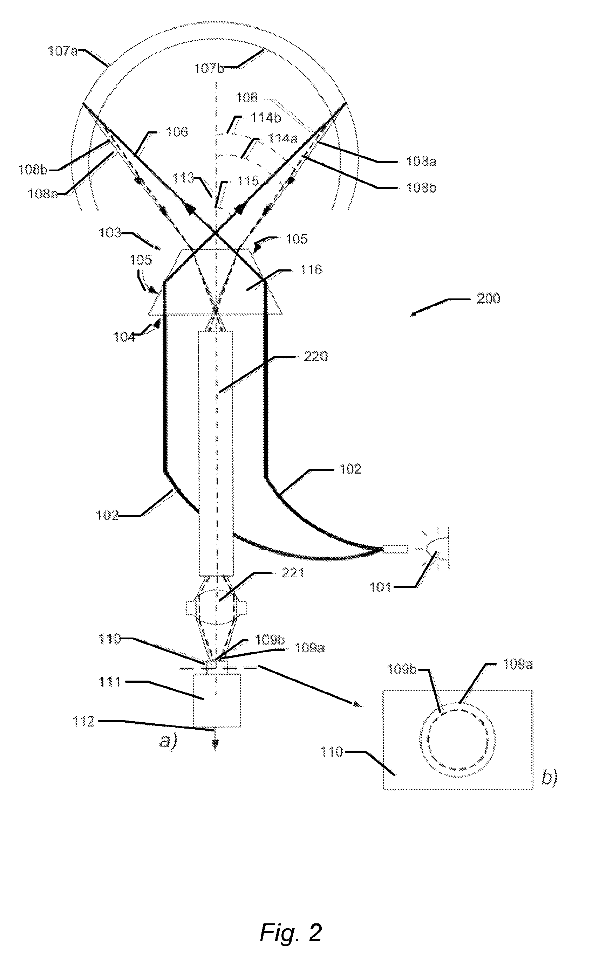

Fig. 2 shows a schematic view of another example of an apparatus, generally designated 200, for obtaining geometrical data relating to the internal surface of a cavity. The apparatus 200 is similar to the apparatus described in connection with 100, and will therefore not be described in detail again here. However, while the light sensitive element 110 of the apparatus shown in fig. 1 was arranged adjacent to the distal end portion 103, such that the reflected light is captured at the distal end of the probe, the light sensitive element 110 of the apparatus 200 is arranged remote from the distal end 103. To this end, the optical system 116 at the distal end portion 103 directs the reflected light into the distal end of a light guide 220, which in the following will be referred to as an imaging guide, the imaging guide 220 thus directs the received light, e.g. via a further lens or lens system 221 to the light sensitive element 110.

While the apparatus of fig. 1 provides a simpler construction, the apparatus 200 does not require a light sensitive element, e.g. a CCD which is small enough to be mounted at the tip of the probe, which is going to enter the cavity. For example, the light guides 102 and the imaging guide 220 may be arranged in a flexible tube connecting the distal end portion 103 with a proximal unit including the light source 110, the detector 110, and, optionally a signal processing unit 111.

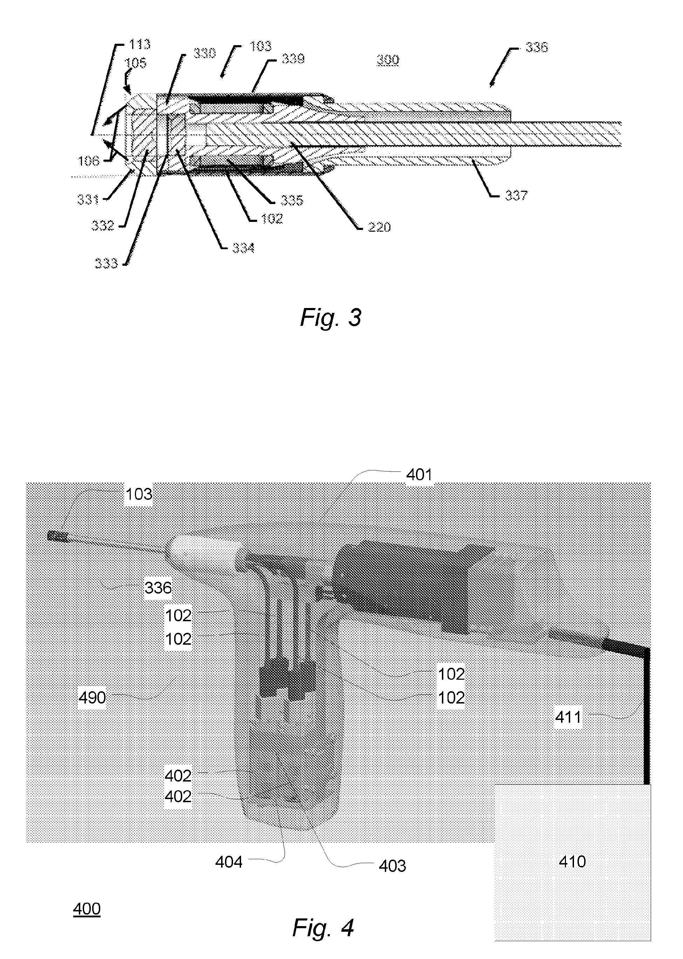

Fig. 3 shows a sectional view of the distal end of a probe of an apparatus for obtaining geometrical data relating to the internal surface of a cavity. The probe, generally designated 300, has a distal light-emitting end portion 103 and a rod portion 336, which connects the distal portion to a proximal part (not explicitly shown). The rod portion 336 comprises a flexible pipe 337, a set of light guides 102 and an image guide 220. The image guide 220 is placed centrally in the pipe 337, and the light guides 102 are arranged between the pipe 337 and the image guide 220. Near the tip of the probe the image guide 220 and the light guides 102 are connected to a bushing 330 surrounded by an outer tube 339.

The probe further comprises an optical system including an annular lens 331 arranged at the bushing 330 to capture the light emitted from the light guides 102 and to direct the light towards a cavity wall, e.g. as a collimated or focused light beam 106, at an acute angle from the longitudinal axis of the probe which also defines the optical axis 113 and the direction of insertion.

Light reflected from the cavity wall will enter the tube 339 through a central receiving lens 332 of the optical system. From the lens 332 the light is directed via an aperture 333 and a further imaging lens 334 towards a surface of the image guide 220. The aperture 333 increases the depth of field and prevents stray light from reaching the sensor. The light received on the surface of the image guide 220 is transmitted through the image guide 220, and will appear at the other end thereof. Here the image is captured by a CCD array (not shown). The signal from the CCD is transferred to a signal processing unit for further processing in order to calculate the distance from the probe to the canal wall. This is done by a triangulation method well known as such in the art.

In general, even though the light 106 directed towards the cavity wall may be unfocussed or uncollimated, the use of focused or collimated light provides better contrast and thus results in a more precise detection of the distance between the probe and the cavity wall.

The probe 300 further comprises one or more coils 335 used to generate a magnetic field, which is picked up by sensors arranged outside the cavity so as to determine the position of the probe relative to the external sensors. Thus, when the sensors are arranged in a fixed spatial relationship to the cavity, a signal/data processing unit can compute spatial coordinates of positions on the inner surface of the cavity from the optical distance measurements relative to the probe and from the position measurements of the position of the probe relative to the external sensors.

In the above embodiments, the light guides and the image guide have been shown as separate light guides. It will be appreciated that alternatively a single light guide may be used for both directing light to the tip of the probe and for transmitting the reflected light back to the CCD element. Such a combined guide may require an additional beam splitter, and may further have the disadvantage of a reduced signal to noise ratio.

Hence, in the above, a probe of an apparatus has been disclosed which is suitable for obtaining geometrical data of the inner surface of a cavity, such as an ear canal.

Fig. 4 shows a probing device 400 comprising a handheld probe body 490 and a power supply 410. The handheld probe body 490 may be connected to the power supply 410 via an electric cable 411. Alternatively, the handheld probe body 490 may be connected to a battery 410 via an electric cable 411. Alternatively, the battery 410 may be rigidly connected to the handheld probe body 490 e.g. by being contained in said handheld probe body 490. The power supply 410 and/or battery 410 may supply the probing device 400 with electrical power.

The handheld probe body 490 may comprise a distal light-emitting end portion 103 and a rod portion 336 which connects the distal light-emitting end portion 103 to a proximal part 401. The distal light-emitting end portion 103 is rigidly fixed to the rod portion 336 and the rod portion 336 is rigidly fixed to the proximal part 401 e.g. by use of glue or screws or any other means of rigidly fixing.

The proximal part 401 may be rigidly fixed in connection with a number of light sources 402 such as one or more laser sources. In an embodiment, the proximal part 401 may be rigidly fixed in connection with four laser diodes 402. The laser diodes may, for example, emit electromagnetic radiation such as light chosen in the wavelength range 400nm - 480nm. In an embodiment, the proximal part 401 comprises four laser diodes emitting green light i.e. light with a wavelength of approximately 532nm. In an alternative embodiment, the proximal part 401 comprises four laser diodes emit blue light with a wavelength of approximately 473nm. In an alternative embodiment, the proximal part comprises two green laser diodes and two blue laser diodes. In an alternative embodiment, the proximal part 401 comprises one or more laser sources emitting light at a wavelength chosen in the visible spectrum i.e. in the range from approximately 400nm to approximately 750nm.

A number of light sources 402, e.g. four laser diodes, may be rigidly fixed in connection with the outside of the proximal part 401 e.g. using glue to attach the laser sources 402 to the outside of the proximal part 401. Alternatively or additionally, the four laser diodes 402 may be fixed to the outside of the proximal part 401 by one or more screws for each of the four laser diodes 402 which in this way are fixed to the outside of the proximal part 401.

Alternatively or additionally, a number of light sources 402 may be contained in the proximal part 401. For example, if the proximal part 401 contains four laser diodes, then the four laser diodes 402 may be contained in the proximal part 401 e.g. by glue to one or more inside walls of the proximal part 401. Alternatively or additionally, the four laser diodes may be fixed to the inside of the proximal part by one or more screws for each of the four laser diodes 402 contained in the proximal part 401.

The handheld probe body 490 may further comprise a number of light guides 102 such as one or more optical fibers. In an embodiment, the handheld probe body comprises a light guide 102 for each of the light sources 402 rigidly fixed in connection with the proximal part 401. For example, if the handheld probe body 490 comprises four laser diodes 402, then the handheld probe body 490 may further comprise four optical fibers 102. One or more of the optical fibers may, for example, be single mode optical fibers. Alternatively or additionally, one or more of the optical fibers may be multimode optical fibers. Alternatively, the proximal part 401 may comprise one or more single mode fibers and one or more multimode fibers.

A first end of each of the light guides 102 may be optically coupled to the rear surface 104 of the optical system 116 in the distal light-emitting end portion 103. For example, the first end of each of the light guides 102 may be glued to the optical system 116 via an optical adhesive. For example, if the handheld probe body 490 comprises four multimode optical fibers, the first end of each of the four multimode optical fibers may be glued to the rear surface 104 of the optical system 116. Alternatively or additionally, the optical system 116 may comprise one or more recesses such that the first end of each light guide 102 may be frictionally coupled to said optical system 116.

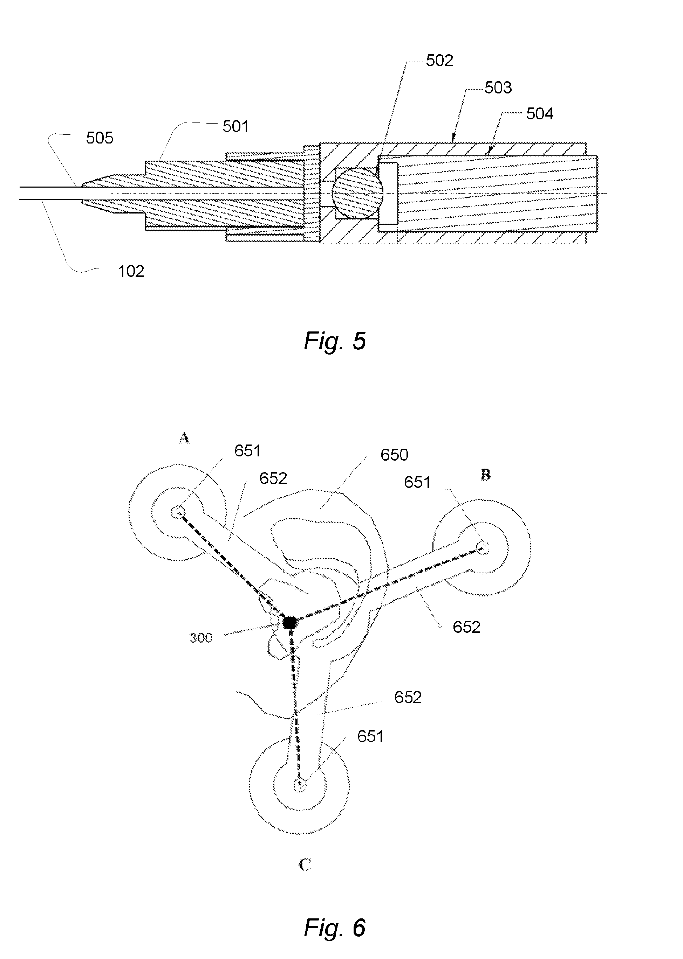

A second end of each of the light guides 102 may be optically coupled to a laser diode 402. For example, the second end of each of the light guides 102 may be glued to a respective one of the one or more light sources 402. For example, if the handheld probe body 490 comprises four laser diodes 402 and four multimode optical fibers 102, then the second end of each of the four multimode optical fibers may be glued to a respective one of each of the four laser diodes 402. Alternatively or additionally, the second end of each of the four multimode optical fibers may be frictionally coupled to respective ones of the four laser diodes 402 e.g. via a frictional coupling as shown in Fig. 5.

By rigidly fixing the light sources 402 in connection with the handheld probe body 490, it is ensured that the optical fibers are substantially stationary with respect to the laser diodes 402 and the rear surface 104 of the optical system 116 i.e. that the optical fibers are substantially stationary with respect to the handheld probe body 490. Thereby, it is achieved that when a user moves the handheld probe body 490 e.g. during a scan of one or more internal surfaces of a cavity, then the optical fibers may remain substantially stationary with respect to the handheld probe body 490 and thus movement determined loss in the optical fibers due to the movement of the handheld probe body 490 may be reduced and/or avoided.

In an embodiment, the light guides 102 may be rigidly fixed in connection with said handheld probe body 490. For example, the light guides 102 may be glued, along a longitudinal axis of the light guides, to one or more internal surfaces of the handheld probe body 490 such that the position of the light guides 102 is fixed with respect to the handheld probe body 490.

In an embodiment, the proximal part 401 comprises a heat sink 403. The one or more light sources 402 may be thermally connected to said heat sink 403 such that heat generated by the light sources may be dissipated to and/or absorbed by the heat sink 403. The thermal contact between the one or more light sources 402 and the heat sink 403 may, for example, be made by physically contacting said one or more light sources 402 to said heat sink.

The heat sink 403 may, for example, comprise an aluminum box. In an embodiment, the one or more light sources may be contained in said aluminum box. Alternatively or additionally, the heat sink 403 may comprise a Peltier cooler. By way of the heat sink, surplus heat from the light sources 402 is dissipated such that hot spots on the surface of hand held probe body 490 is avoided. This allows the user to grip the hand held device 490 without subjecting his hand to uncomfortable or harmful heat.

In an embodiment, the proximal part 401 further comprises a printed circuit board (PCB) 404 for controlling the one or more light sources 402. For example, the PCB may control the electrical power to the one or more light sources 402. Thereby, the PCB may, for example control the modulation of the optical output from the one or more light sources 402 and/or the PCB 404 may control the power of the optical output signal from the one or more light sources 402.

The PCB may be thermally coupled to the heat sink. For example, the PCB may be contained in the heat sink. In the case the heat sink is made of aluminum or another metal, the PCB may further be shielded from electromagnetic noise from other electromagnetic sources such as for example, the coils 335 generating a magnetic field. Additionally, the electromagnetic noise generated by the PCB is further shielded such as not to interfere with e.g. the magnetic field generated by the one or more coils 335.

Fig. 5 shows an embodiment in which the second end 505 of a light guide 102 is connected to a light source 504 via a connector 501 and a housing. The connector 501 may, for example, be chosen from the group consisting of SC optical connector, MU optical connector, ST optical connector, LC optical connector, FC optical connector and SMA optical connector.

The second end 505 of a light guide 102 comprising a connector 501 may be frictionally coupled to a light source, e.g. a laser diode, 504 contained in a housing 503. For example, the laser diode 504 may be contained in the housing 503. The connector 501 may, for example, be frictionally coupled to the housing 503. Alternatively or additionally, the connector 501 may comprise a thread suitable to be coupled to a similar thread on the housing 503. The housing 503 may further comprise a lens 502 enabling light from the light source 504 to be optically coupled into the light guide 505 e.g. with a low loss.

By coupling the second end 505 of a light guide 102 to a light source 402, 504 via a connector 501 and housing 503, enables easy replacement of the light source 402, 504 or the probe tip 103 and one or more light guides 102.

Fig. 6 is a side view showing the human ear and illustrating the use of the apparatus described herein. In use the probe 300, 400 is gently inserted into the ear 650, and magnetic sensors 651 are placed in close relation to the patient's head. Placing the probe in the ear is done while objects in front of the probe are monitored as described above. A real picture may be obtained, and/or the distance to the tympanic membrane is measured as described herein. The picture captured this way is displayed on a monitor, such that the operator may know when the probe is approaching the tympanic membrane. Once the region near the tympanic membrane is reached, the measurements may commence. This may be done while retracting the probe as corresponding values of the distances to the canal wall and the position of the probe are recorded. The recording is continued until the probe reaches the outer regions of the outer ear.

In the example of fig. 5, at each sensor position A, B and C two or more sensors 651 are located, which are designed to register the magnetic field in each their direction. Through this arrangement the exact location and orientation of the tip of the probe can be determined at any time. In the case shown in fig. 6, the probe 300, 400 is located inside the canal of a human ear 650, shown schematically in the figure. The three sensor locations are arranged in a fixed construction, which in use is held immobilized relative to the patient's head. In the embodiment shown in fig. 6, the fixed construction comprises a tripod, whereby each of the sensor positions are placed at the outer end of each of the branches 652 of the tripod. In use, a coil at the tip of the probe is driven at a fixed frequency and by using a lock-in procedure, thereby allowing any noise coming from other magnetic fields in the surroundings to be cancelled out from the sensor signals. The tripod shown in fig. 5 is only indicated schematically in the figure. When the sensors A, B and C are used in reality, they will be arranged in a fixture, which leaves the ear canal open for insertion of the probe.

A guiding arrangement for the probe may be located at the tripod shown in fig. 6. A guiding arrangement may comprise two or more opposite flat rollers between which the probe is to pass. By slightly squeezing the probe between the rollers, rotational movement of the probe around the length direction is prevented. Such a guiding mechanism may however be implemented in many different ways.

Although some embodiments have been described and shown in detail, the invention is not restricted to them, but may also be embodied in other ways within the scope of the subject matter defined in the following claims. In particular, it is to be understood that other embodiments may be utilized and structural and functional modifications may be made without departing from the scope of the present invention.

In device claims enumerating several means, several of these means can be embodied by one and the same item of hardware. The mere fact that certain measures are recited in mutually different dependent claims or described in different embodiments does not indicate that a combination of these measures cannot be used to advantage.

It should be emphasized that the term "comprises/comprising" when used in this specification is taken to specify the presence of stated features, integers, steps or components but does not preclude the presence or addition of one or more other features, integers, steps, components or groups thereof.

## Claims

1. A probing device for obtaining geometrical data related to a cavity, the probing device comprising
• at least one light source for generating electromagnetic radiation;
• a handheld probe body comprising a probe tip for obtaining said geometrical data related to a cavity;
• at least one light guide for guiding electromagnetic radiation; wherein a first end of said at least one light guide is optically coupled to said probe tip; and
• wherein the at least one light source is optically coupled to a second end of said at least one light guide and wherein the at least one light source is rigidly connected to said probe body.

2. A probing device according to claim 1, wherein the at least one light source is contained in said probe body.

3. A probing device according to claim 1 or claim 2, wherein the at least one light source is thermally coupled to a heat sink.

4. A probing device according to anyone of claims 1 to 3, wherein the handheld probing device further comprises a control circuit for controlling said at least one light source.

5. A probing device according to claim 4, wherein the control circuit is thermally coupled to and contained in a heat sink.

6. A probing device according to anyone of claims 1 to 5, wherein the at least one light source comprises at least one laser emitting electromagnetic radiation at a wavelength chosen in the interval 400nm - 480nm.

7. A probing device according to anyone of claims 1 to 6, wherein the at least one light guide comprises at least one multimode fiber.

8. A probing device according to anyone of claims 1 to 7, wherein each of the at least one light guides comprises a connector at their second end for optically coupling respective ones of said at least one light guides to respective ones of the at least one light sources via respective housings.

9. A probing device according to claim 8, wherein the connector is chosen from the group consisting of SC optical connector, MU optical connector, ST optical connector, LC optical connector, FC optical connector and SMA optical connector.

10. A system for obtaining geometrical data related to a cavity, the system comprising at least one probing device according to anyone of claims 1 to 9 and the system further comprising at least a plurality of magnetic sensors for detecting the at least one probing device.
